# EUROPEAN PATENT SPECIFICATION

(11) **EP 0 520 618 B1**
(45) Date of publication and mention of the grant of the patent: **02.01.1997**
(21) Application number: 92304736.9
(22) Date of filing: 26.05.1992
(51) Int. Cl.: A61M 5/28, A61M 5/31, A61M 5/32

(54) **Dual chamber prefillable syringe**
Vorgefüllte Zweikammer-Spritze
Seringue préremplie à double compartiment

(30) Priority: 26.06.1991 JP 57218/91 U
(43) Date of publication of application: 30.12.1992
(73) Proprietor: ARTE CORPORATION, Chiyoda-ku, Tokyo (JP)
(72) Inventor: Takamura, Noriyuki, Iwaki-Shi, Fukushima (JP)
(74) Representative: Crouch, David John

(56) References cited:
- WO-A-88/02265
- FR-A- 1 099 362
- US-A- 4 254 768

## Description

This invention relates to a dual chamber prefillable syringe comprising,
a syringe outer cylinder having a bypass groove recessed longitudinally thereof on the inner wall substantially at the centre thereof,
a sealing plug of thickness shorter than the length of the bypass groove and liquid-tightly sealed at a position near an outer cylinder opening from the bypass groove to form a first inner chamber between a liquid-tightly sealed cylindrical end thereof and the sealing plug,
a plunger liquid-tightly sealed at a position near the outer cylinder opening from the sealing plug to form a second inner chamber between the plunger and the sealing plug,
the syringe outer cylinder being split in a direction perpendicular to a longitudinal direction at a position near the outer cylinder opening from the bypass groove to be divided into a front chamber outer cylinder and a rear chamber outer cylinder,
the front chamber outer cylinder including the bypass groove, the cylindrical end and a first split opening,
the rear chamber outer cylinder including the outer cylinder opening and a second split opening,
an inner peripheral edge of the split opening being opposed over an entire periphery to an inner peripheral edge of the second split opening, so that the front chamber outer cylinder and the rear chamber outer cylinder are disposed in series, and
the front chamber outer cylinder and the rear chamber outer cylinder being liquid-tightly held by a coupling component.

Thus predetermined quantities of first and second medicines can be liquid-tightly partitioned in a syringe outer cylinder in a sterilized state for injecting both the medicines in mixture in one chamber as a parenteral solution.

Unstable medicine which cannot be stocked as parenteral solution is generally powdered by lyophilizing by a freeze-drying apparatus as powdered medicine. The powdered medicine and liquid medicine for dissolving the medicine are sealed in a sterilized state and stocked, mixed at the time of injection and used as a parenteral solution.

When the powder medicine and liquid medicine are used, a predetermined quantity of liquid medicine sealed in an ampule or a vial is first sucked in a syringe in which a syringe needle is mounted. Then, the needle is pierced into a rubber plug of a vial in which a predetermined powdered medicine is sealed, the liquid medicine in the syringe is poured in the vial to mix the powdered medicine with the liquid medicine as a parenteral solution, the parenteral solution is again sucked into the syringe, the needle is removed from the rubber plug of the vial, and injected into a patient as it is. However, this operation is complicated and may take a long time. There is a danger of imparting pain to the patient at the time of injecting due to the slight deformation of the end of the needle to be pierced into the rubber plug of the vial at the time of piercing. Further, there is also a danger that the part of the rubber plug of the vial is cut out by a pierced syringe needle as finely pulverized rubber pieces which are, in turn introduced into the needle and hence injected together with the parenteral solution in vivo into a patient and also a danger that the needle and/or the parenteral solution are contaminated with bacteria or foreign matter in the atmosphere or adhering to the rubber plug.

In order to eliminate the above dangers, there has been invented a conventional dual chamber prefillable syringe in which predetermined quantities of powdered medicine and liquid medicine are liquid-tightly partitioned in one syringe in a sterilized state.

For example, in FR-A-1,099,362 there is described a syringe having the construction set out in the opening paragraph of the present specification, in which a first chamber contains an antibiotic, a vitamin, a hormone or a biological product in powder form which only retains its therapeutic properties whilst it remains dry, and a second chamber which contains a liquid, the contents of the two chambers not being mixed until the moment of injection into the patient.

Further examples of conventional dual chamber prefillable syringes are described in Japanese Utility Model Publication No. 49-14465 in Figure 11 and in Japanese Patent Application Laid-open No. 62-5357 in Figure 12. As shown in Figures 11 and 12, in both the dual chamber prefillable syringes, bypass grooves 1c, 2c are recessed on the inner walls 1b, 2b substantially at the centres of the syringe outer cylinders 1a, 1a longitudinally of the syringe outer cylinders 1a, 2a. Sealing plugs 1f, 2f having smaller thicknesses 1e, 2e than the lengths 1d, 2d of the bypass grooves 1c, 2c are liquid-tightly engaged with positions near outer cylinder openings 1g, 2g from the bypass grooves 1c, 2c in the syringe outer cylinders la, la. First inner chambers 1j, 1j are formed between the cylinder ends 1i, 2i liquid-tightly sealed by a sealing plug h or a stopper t and the sealing plugs 1f, 2f. Plungers 1k, 2k are liquid-tightly engaged at positions near the outer cylinder openings 1g, 2g from the sealing plugs 1f, 2f to form second inner chambers 1m, 2m between the plungers 1k, 2k and the sealing plugs 1f, 2f in the same configurations.

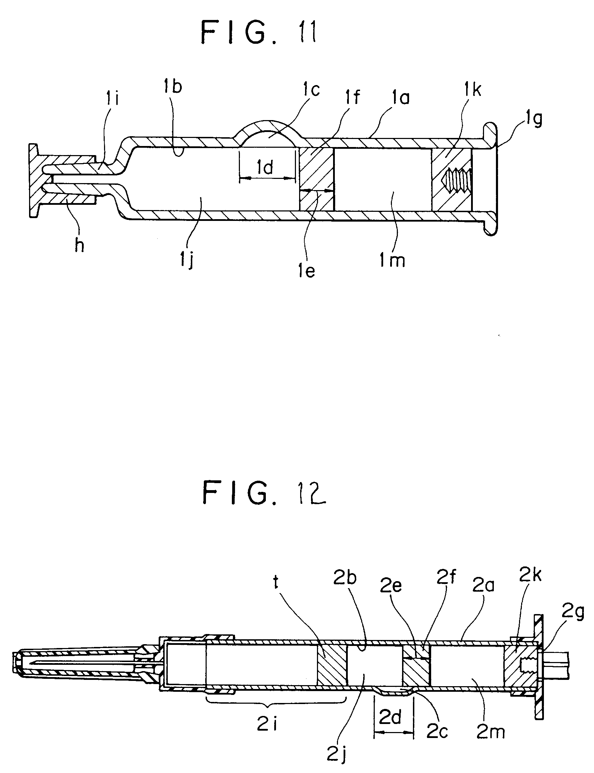

Since the dual chamber prefillable syringes shown in Figures 11 and 12 are constructed in the same configurations as described above, the following prior art will be described with reference to the dual chamber prefillable syringe shown in Figure 11.

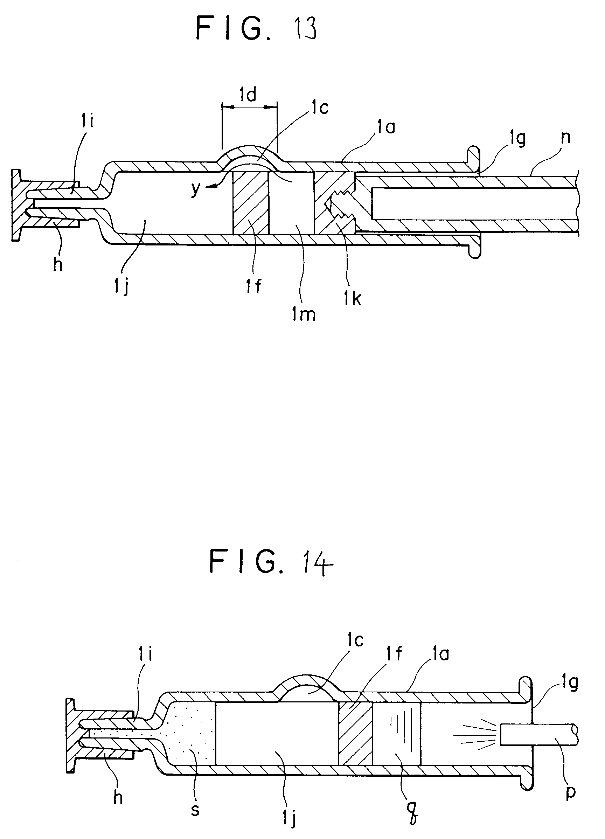

When the dual chamber prefillable syringe in which powdered medicine of a predetermined quantity is sealed in a sterilized state in the first inner chamber 1j and liquid medicine of a predetermined quantity is sealed in a sterilized state in a second inner chamber 1m is used, as shown in Figure 13, the plunger 1k is pushed forward by a syringe inner cylinder n to raise the hydraulic pressure of the liquid medicine, thereby pressing forward the sealing plug 1f to the bypass groove 1c side of the syringe outer cylinder la by means of the raised hydraulic pressure. When the plug 1f is thus pressed and introduced to a range of the length 1d of the bypass groove 1c, the first inner chamber 1j communicates with the second inner chamber 1m so that the inner pressures in both the inner chambers 1j and 1m become uniform, and the plug 1f is hence stopped. When the plunger k is further pressed into the syringe inner cylinder n, the liquid medicine is fed into the first inner chamber 1j through the bypass groove 1c as indicated by an arrow y in Figure 13, the plunger 1k contacts the plug 1f, the entire liquid medicine is fed into the first inner chamber 1j, and the liquid medicine is mixed with the powdered medicine in the first inner chamber 1j to produce a parenteral solution.

Since both the powdered medicine and the liquid medicine filled in advance and sealed in the syringe outer cylinder 1 are injected in vivo into a patent as a parenteral solution, they are not contaminated with bacteria, etc., at all. Therefore, it is best if the powdered medicine is sealed in the first inner chamber 1j, the liquid medicine is sealed in the second inner chamber 1m and the dual chamber prefillable syringe in which both the powdered and liquid medicines are filled is then heated to be sterilized by high pressure steam, etc., thereby to simultaneously sterilize both the powdered and liquid medicines, but, if the powdered medicine is heat treated, its physical properties are varied. Therefore, it is impossible to heat to sterilize the dual chamber prefillable syringe in which both the powdered and liquid medicines are retained and yet kept apart.

Therefore, in the conventional dual chamber prefillable syringe, liquid medicine is filled in a sterilized state in the syringe outer cylinder 1a, medicine liquid is freeze-dried to powdered medicine by a freeze-drying apparatus held therein in a sterilized state, the sealing plug 1f is engaged at a position near the outer cylinder opening 1g from the bypass groove 1c in the syringe outer cylinder 1a in the freeze-drying apparatus, and the powdered medicine is sealed in a sterilized state in the first inner chamber 1j in the syringe outer cylinder 1a. Thereafter, as shown in Figure 14, liquid medicine q is filled from a sterilized sealed vessel through a pouring pipe p into the syringe outer cylinder 1a in which powdered medicine s is sealed in a sterilized state in the first inner chamber 1j, the plunger 1k is subsequently sealed at a position near the outer cylinder opening 1g in the syringe outer cylinder 1a, and the liquid medicine q is sealed in a sterilized state in the second inner chamber 1m in the syringe outer cylinder 1a.

The filling and sealing of the liquid medicine q in the syringe outer cylinder 1a in which the powdered medicine s is sealed in a sterilized state after the powdered medicine s is sealed in the sterilized state is conducted under a sterilized environment, but the working steps are considerably long. Therefore, there has been a danger that the liquid medicine q, the powdered medicine s and the syringe outer cylinder 1a are contaminated due to ingress of bacteria, etc., during the filling and sealing in the conventional dual chamber prefillable syringe.

As described above, the powdered medicine s is sealed in the sterilized state in the first inner chamber 1j in the syringe outer cylinder 1a. To this end, after the medicine liquid to be used as powdered medicine s of a predetermined quantity is filled in a sterilized environment in the syringe outer cylinder 1a, a freeze-drying process of the medicine liquid to powdered medicine s in a freeze-drying apparatus of a sterilized environment and a sealing process of inserting a sealing plug 1f into the syringe outer cylinder 1a at a position near the outer cylinder opening 1a via the bypass groove 1c by an insertion rod set in the freeze-drying apparatus are executed.

Since it takes 20 hours or longer to carry out the freeze drying work in the freeze-drying apparatus for conducting both the freeze-drying process and the sealing process, it is important to improve the production efficiency of the procedures so as to improve the production efficiency of the entire procedure. In order to improve the production efficiency of the freeze-drying procedure, at is required to set as many syringe outer cylinders 1a sealed at the cylinder ends in the freeze-drying apparatus as is possible.

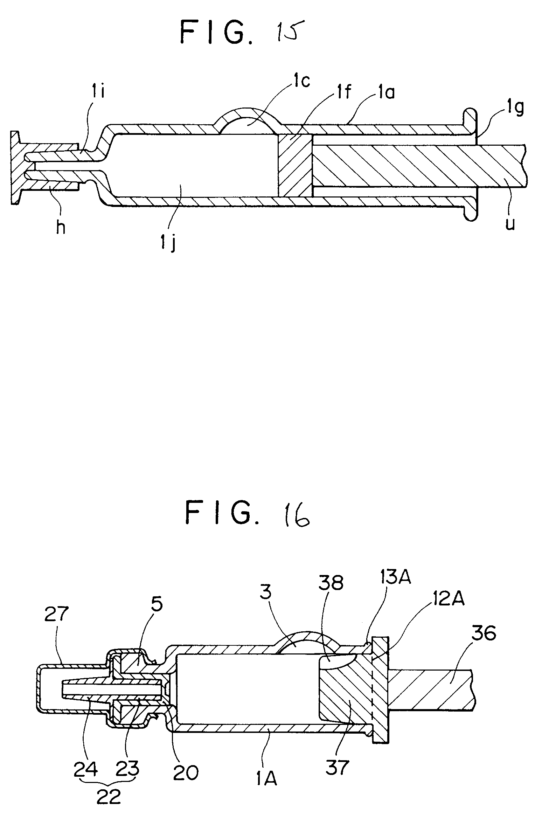

However, since the syringe outer cylinder 1a is long in the conventional dual chamber prefillable syringe, the number of the syringe outer cylinders 1a which can be set in the freeze-drying apparatus is limited. Since the sealing position of the sealing plug 1f is disposed substantially at the centre of the outer cylinder 1a, the insertion rod u for inserting the sealing plug 1f to the sealing position becomes long as shown in Figure 15, and the space in the freeze-drying apparatus occupied by the insertion rod u is increased, and hence there occurs a problem that the number of the outer cylinders 1a which can be set in the freeze-drying apparatus is limited.

### SUMMARY OF THE INVENTION

An object of this invention is to provide a dual chamber prefillable syringe which obviates the above-described problems of the prior art as follows:
(a) When a predetermined quantity of powdered medicine is sealed in a sterilized state in a first inner chamber and a predetermined quantity of liquid medicine for dissolving the powdered medicine is sealed in a sterilized state in a second inner chamber, the parallel process of sealing the powdered medicine and the liquid medicine is performed, filling and sealing procedures of the liquid medicine in a syringe outer cylinder in which the powdered medicine is sealed in a sterilized state after the powdered medicine is sealed in a sterilized state are eliminated, and the danger that the powdered medicine, the liquid medicine and the syringe outer cylinder are contaminated with bacteria, etc., is eliminated.
(b) In a freeze-drying work for freeze-drying medicine liquid filled in the syringe outer cylinder to powdered medicine and sealing the resultant powdered medicine in a sterilized state, the number of syringe outer cylinders which can be set in a freeze-drying apparatus is increased to improve production efficiency of the freeze-drying work.

Accordingly, the present invention is directed to a dual chamber prefillable syringe having the construction set out in the opening sentence of the present specification, in which the sealing plug comprises a first sealing plug and a second sealing plug by splitting the sealing plug in a direction perpendicular to a thickness direction,
the first sealing plug is liquid-tightly sealed at a position near the first split opening in the front chamber outer cylinder,
the second sealing plug is liquid-tightly sealed at a position near the second split opening of the rear chamber outer cylinder, and
a distance from an end face of the first sealing plug at the bypass groove side to an end face of the second sealing plug at the outer cylinder opening side is set shorter than the length of the bypass groove.

Preferably, the dual chamber prefillable syringe further comprises a needle mounting cylinder including a base cylinder and an end cylinder, a bottomed hollow guard, and a bottomed hollow sealing plug made of an elastic material,
the cylindrical end of the front chamber outer cylinder is formed in a wide port bottle shape,
the bottomed hollow sealing plug is sealed in the cylindrical end in such a manner that a bottom of the bottomed hollow sealing plug is faced towards the first inner chamber, so that the cylindrical end is liquid-tightly sealed,
the base cylinder of the needle mounting cylinder is sealed in a hollow section of the sealing plug,
the end cylinder of the needle mounting cylinder protrudes from an end opening of the cylindrical end,
an outer peripheral surface of the end cylinder is gradually reduced in diameter toward its end as a tapered surface,
a cylindrical hole is perforated through the needle mounting cylinder from an end face of the end cylinder to an end face of the base cylinder,
a guard base of the bottomed hollow guard is gas-tightly secured to an outer wall of the cylindrical end so that the end cylinder of the needle mounting cylinder is sealed in the guard, and
a collapsible brittle portion is circumferentially formed at a position of widest diameter portion of the tapered surface.

Advantageously, the dual chamber prefillable syringe further comprises a stopper made of an elastic material, a bottomed hollow end housing and a bottomed hollow cap having an elasticity,
the stopper is sealed in the cylindrical end of the front chamber outer cylinder to liquid-tightly seal the cylindrical end,
a distance from the end opening of the cylindrical end to the stopper is set longer than a distance from the bypass groove to an end face of the plunger at the bypass groove side,
an end of the cylindrical end is gas-tightly engaged within the coupling portion of the bottomed hollow end housing, so that a hollow section of the end housing from the end opening of the cylindrical end to an inner surface of a bottom of the end housing is formed as a bypass chamber,
a length of the bypass chamber is set longer than a thickness of the stopper,
the end opening is included in a surface which contacts the bypass chamber,
a plurality of bypasses are recessed longitudinally of the end housing on an inner wall of the end housing,
a plurality of bottom grooves coupled to the bypass is recessed radially on an inner surface of the bottom of the end housing,
a housing recess is formed at a portion concentrated with all bottom grooves to connect all the bottom grooves to the housing recess,
a needle mounting portion is suspended from an outer surface of the bottom of the housing,
a small hole from an end of the needle mounting to the housing recess is perforated through the needle mounting portion, and
the needle mounting portion is liquid-tightly sealed with the hollow section of the bottomed hollow cap.

In this case, it is desirable that an outer wall surface of the needle mounting portion is gradually reduced in diameter toward its end as a tapered surface, and
an inner wall surface of the bottomed hollow cap is formed in the same tapered surface as the tapered surface.

Also, the syringe may further comprise a needle tube and a cap in which the dual chamber prefillable syringe further comprises a needle tube and a cap,
the outer wall surface of the needle mounting portion is gradually reduced in diameter toward its end in a tapered surface,
a base needle of the needle tube is perforated through from the end of the small hole of the needle mounting portion to the housing recess, so that the base needle is gas-tightly secured to the small hole of the needle mounting portion,
a needle hole perforated through the needle tube is connected to the housing recess,
an end needle of the needle tube is provided to protrude from the needle mounting portion,
the cap acts as a needle guard,
a length of a hollow section of the needle guard is set longer than a total length of the length of the end needle and the length of the needle mounting portion,
an inner wall of the needle guard is formed in the same tapered surface as the tapered surface of the needle mounting portion, and
the end needle is sealed in the hollow section of the needle guard.

The syringe may further comprise a lock having a columnar portion, in which the lock is suspended from the outer surface of the housing bottom,
the columnar portion includes the needle mounting portion therein,
female threads are formed on an inner peripheral surface of the columnar portion,
a flange is formed on an outer wall surface of the cap, and
the flange is engaged by the female thread with the lock.

With a given embodiment of this invention, when a predetermined quantity of the powdered medicine is sealed in a sterilized state in the first inner chamber, a predetermined quantity of the liquid medicine is sealed in a sterilized state in the second inner chamber, and the plunger is pressed forward in the syringe inner cylinder during an injection procedure, the hydraulic pressure of the liquid medicine is raised by pressing forward the plunger, and the sealing plunger is pressed forward to the bypass groove side by the raised hydraulic pressure of the liquid medicine. Thus, when the sealing plug has moved within a range of the length of the bypass groove, the first inner chamber communicates with the second inner chamber through the bypass groove so that the pressures of both the first and second inner chambers become uniform, and hence the sealing plug is stopped at the bypass groove, only the plunger is then continuously pressed forward to feed the liquid medicine into the first inner chamber. Then, the plunger contacts the sealing plug to feed the entire quantity of the liquid medicine into the first inner chamber. Thus, when the powdered medicine is mixed with the liquid medicine, a parenteral solution is produced. Therefore, since the sealing state of the powdered medicine and the liquid medicine is maintained even during the step of producing the parenteral solution by the above-described operation, there is no danger of the powdered and liquid medicines being contaminated with bacteria, etc. during that step.

A predetermined quantity of powdered medicine can be sealed in a sterilized state in the front chamber outer cylinder by freeze-drying. Further, the step of sealing a predetermined quantity of the liquid medicine in the rear chamber outer cylinder, thermally sterilizing it, and sealing the liquid medicine in a sterilized state in the rear chamber outer cylinder and the step of sealing the powdered medicine in a sterilized state in the front chamber outer cylinder can be executed simultaneously. Further, the powdered medicine is sealed in a sterilized state in the front chamber outer cylinder, the liquid medicine is sealed in a sterilized state in the rear chamber outer cylinder, the front chamber outer cylinder is coupled in series with the rear chamber outer cylinder through the coupling component in a sterilized environment, and the dual chamber prefillable syringe in which the powdered medicine and the liquid medicine are sealed in a sterilized state can be assembled. More specifically, when a predetermined quantity of the powder medicine is sealed in a sterilized state in the first inner chamber and a predetermined quantity of the powdered medicine for dissolving the powdered medicine is sealed in a sterilized state in the second inner chamber, the sealing of the powdered medicine and the liquid medicine can be effected simultaneously. Thus, the filling and sealing of the liquid medicine in the syringe outer cylinder in which powdered medicine is sealed in a sterilize stated after the powdered medicine is sealed in a sterilized state like a conventional dual chamber prefillable syringe are eliminated. Therefore, there is no danger that the liquid medicine the powdered medicine and the interior of the syringe outer cylinder will be contaminated with bacteria, etc., during the filling and sealing procedures.

Further, since this invention is constituted as described above, the length of the front chamber outer cylinder becomes shorter than that of the syringe outer cylinder of the conventional dual chamber prefillable syringe. In the sterilized freeze-drying apparatus, the a-split opening of the front chamber outer cylinder is sealed, and the powdered medicine can be sealed in a sterilized state in the front chamber outer cylinder. Therefore, in order to seal the powdered medicine in the freeze-drying apparatus, the length of the inserting rod set in the freeze-drying apparatus can be shortened as compared with that of the conventional inserting rod, a space in the freeze-drying apparatus occupied by the inserting rod is reduced, and the working space for setting the front chamber outer cylinder can be increased. Therefore, the length of the front chamber outer cylinder is shortened as compared with that of the conventional dual chamber prefillable syringe, and the number of the front chamber outer cylinders which can be set in the working space can be increased as compared with the conventional dual chamber prefillable syringe to improve the production efficiency of the freeze-drying work by the freeze-drying apparatus.

Even if the sealing plug upon assembly of the dual chamber prefillable syringe is deviated longitudinally of the syringe outer cylinder therein, the thickness of the sealing plug need not be increased in length of the bypass groove as in the case in which the sealing plug is split. Therefore, since the sealing plug is reliably located in a range of the length of the bypass groove, the first inner chamber can effectively communicate with the second inner chamber, and the assembling can be facilitated.

The medicinal liquid filled in the front chamber outer cylinder may be freeze-dried to powdered medicine by the freeze-drying work of the freeze-drying apparatus in which the interior is sterilized, and the powdered medicine can be sealed in a sterilized state in the front chamber outer cylinder between the sealing plunger and the cylindrical end. The liquid medicine is sealed in the rear chamber outer cylinder between the sealed plug and the plunger, and heated to be sterilized to the able to seal the liquid medicine in advance in a sterilized state in the rear chamber outer cylinder. Therefore, the powdered medicine in the front chamber outer cylinder remains sealed in a sterilized state, and the liquid medicine in the rear chamber outer cylinder also remains sealed in a sterilized state, and the dual chamber prefillable syringe in which the powdered medicine and the powdered medicine are sealed can be assembled. Therefore, the prevention of ingress of bacteria in the powdered medicine in the front chamber outer cylinder and the liquid medicine in the rear chamber outer cylinder can be reliably performed, and a danger in which both the medicines are contaminated by bacteria, etc., is reduced.

Since a distance from the end face of the sealing plug at the bypass groove side to the end face of the sealing plug at the outer cylinder opening side is set shorter than the length of the bypass groove, when the plunger is pressed forward to press forward the sealing plug and the sealing plug in a range of the length of the bypass groove, the first inner chamber can be reliably put into communication with the second inner chamber. Therefore, the liquid medicine in the second inner chamber is fed into the first inner chamber to mix the powdered medicine with the liquid medicine in the first inner chamber, thereby producing a parenteral solution.

The end cylinder of the needle mounting cylinder can be sealed in a sterilized state by sterilizing such as heating to sterilize the powdered medicine in the front chamber outer cylinder before the powdered medicine is sealed in the front chamber outer cylinder, and upon injection, side pressure is applied to the guard by a finger head to break the guard at the collapsible brittle portion to expose the end cylinder, thereby mounting a syringe needle on the bare tapered surface of the end cylinder. Therefore, the syringe needle can be easily mounted, and the end cylinder for mounting the syringe needle is held in a sterilized state up to the time of injection.

The needle mounting portion can be held by the cap in a sterilized state by sterilizing such as thermally sterilizing the front chamber outer cylinder before the powdered medicine is sealed in the front chamber outer cylinder, and the interior of the small hole of the needle mounting portion, the bypass chamber and the interior of the front chamber outer cylinder from the stopper to the end opening can be sealed in the sterilized state.

In the case that the powdered medicine is sealed in the first inner chamber and the liquid medicine is sealed in the second inner chamber, upon injection, the cap is removed, and the plunger is pressed forward in the syringe inner cylinder. Then, the hydraulic pressure of the liquid medicine is raised by pressing forward the plunger, the sealing plug is pressed by the raised hydraulic pressure of the liquid medicine, the air in the second inner chamber is compressed by pressing the sealing plug to raise the pneumatic pressure in the second inner chamber to press forward the stopper, but when the cap is removed, the bypass chamber in the end housing communicates with the atmosphere. Accordingly, even if the stopper is pressed, the air in the bypasses is released into the atmosphere, and the internal pressure in the bypass chamber is not raised. Therefore, the sealing plug is smoothly pressed forward to the bypass groove side.

When the plunger is subsequently pressed forward, the sealing plug is pressed in a range of the length of the bypass groove, the first inner chamber communicates with the second inner chamber through the bypass groove, the sealing plug is stopped, only the plunger is thereafter continuously pressed forward to feed the liquid medicine into the first inner chamber, the plunger thus contacts the sealing plug, the entire quantity of the liquid medicine is fed into the first inner chamber, and the powdered medicine and the liquid medicine are mixed in the first inner chamber, thereby producing a parenteral solution. In this case, the stopper is also pressed by pressing the plunger, but since the distance from the end opening of the front chamber outer cylinder to the stopper is set longer than the distance from the bypass groove to the end face of the plunger at the bypass groove side, the stopper is not pressed into the bypass chamber until the entire quantity of the liquid medicine is fed into the first inner chamber. After the parenteral solution is produced, when the stopper is further pressed into the bypass chamber, the stopper is pressed into the bypass chamber. Thus, when the stopper is introduced into the bypass chamber, the stopper contacts the housing bottom, the parenteral solution is fed from the bypasses of the inner wall of the housing to the bottom groove coupled to the bypasses, introduced to the housing recess coupled to the bottom groove, further fed through the small hole of the needle mounting portion perforated to the recess, and fed externally of the dual chamber prefillable syringe. The sealing plug thus contacts the stopper to press externally the entire quantity of the parenteral solution.

Mounting of the syringe needle on the lure tapered surface of the needle mounting portion can be made easy.

The labour involved in mounting the syringe needle at the needle mounting portion can be eliminated, and the needle mounting portion, the interior of the needle hole of the needle tube, the bypass chamber, the interior of the front chamber outer cylinder from the stopper to the end opening and the end needle of the needle tube can be sealed in sterilized state by the needle guard by sterilizing such as thermally sterilizing the front chamber outer cylinder before the powdered medicine is sealed in the front chamber outer cylinder. Further, the end needle of the needle tube can be protected by the needle guard to prevent the deformation of the end of the end needle. Therefore, the danger of pain being imparted to a patient at the time of injection due to the deformation of the end of the end needle can be eliminated.

The sealing state of the needle mounting portion with the cap can be held by engaging the flange of the cap with the female threads of the lure lock. Thus, there is no danger of the cap being removed from the needle mounting portion up to the time of injection. Therefore, the needle mounting portion, the interior of the small hole of the needle mounting portion, the bypass chamber and the interior of the front chamber outer cylinder from the stopper to the end opening are reliably held in a sterilized state by the cap up to the time of injection.

### BRIEF EXPLANATION OF THE DRAWINGS

Examples of syringes made in accordance with the present invention will now be described with reference to the accompanying drawings, in which :
- Figure 1: is a longitudinal sectional view of a first embodiment of the present invention;
- Figure 2: is a sectional view take along the line W-W of Figure 1;
- Figure 3: is a longitudinal sectional view showing the state of the initial period of pressing a plunger in the syringe shown in Figure 1;
- Figure 4: is a longitudinal sectional view partly omitted showing the state that a double flank syringe needle is mounted in the syringe shown in Figure 1;
- Figure 5: is a longitudinal sectional view of a second embodiment of the present invention;
- Figure 6: is a sectional view taken along the line Y-Y of Figure 5;
- Figure 7: is a longitudinal sectional view showing the state of the initial period of pressing a plunger in the syringe of Figure 5;
- Figure 8: is a longitudinal sectional view partly omitted showing a syringe needle mounted in the syringe of Figure 5;
- Figure 9: is a longitudinal sectional view of a third embodiment of the present invention;
- Figure 10: is a longitudinal sectional view of a fourth embodiment of the present invention;
- Figure 11: is a longitudinal sectional view showing an example of prior art;
- Figure 12: is a longitudinal sectional view of another example of prior art;
- Figure 13: is a longitudinal sectional view showing the state of the initial period of pressing a plunger shown in Figure 11;
- Figure 14: is a longitudinal sectional view showing the state of a liquid medicine filling step in Figure 11;
- Figure 15: is a longitudinal sectional view of the state in which a sealing plunger is inserted in the syringe of Figure 11; and
- Figure 16: is a longitudinal sectional view showing the inserted state of a sealing rubber plug in a front chamber outer cylinder of Figure 1.

### DESCRIPTION OF THE PREFERRED EMBODIMENTS

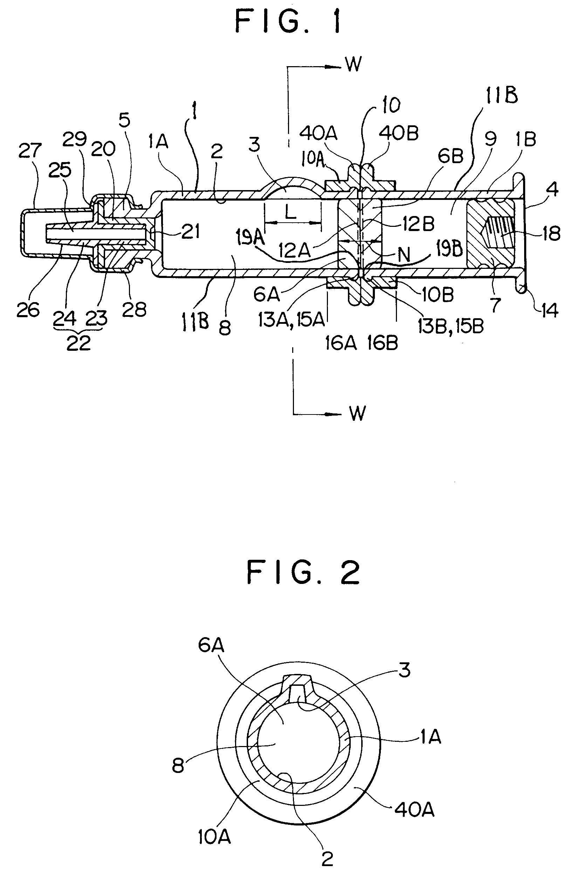

A first embodiment of this invention will be described with reference to Figures 1 to 4 and Figure 16.

A syringe outer cylinder 1 of a cylindrical shape made of glass is formed with a bypass groove 3 longitudinally of the outer cylinder 1 on an inner wall 2 substantially at the centre of the cylinder 1. The outer cylinder a is split by the bypass groove 3 in a direction perpendicular to the longitudinal direction at a position near the outer cylinder opening 4, into a front chamber outer cylinder 1A and a rear chamber outer cylinder 1B. The front chamber outer cylinder 1A has the bypass groove 3, a cylinder end 5 and a first split opening 12A. The rear chamber outer cylinder 1B has the outer cylinder opening 4 and a second split opening 12B. A protrusion 13A is circumferentially formed on an outer wall 11A of the front chamber outer cylinder 1A at the side of the split opening 12A. A protrusion 13B is circumferentially formed at the end of an outer wall 11B of the rear chamber outer cylinder 1B at the side of the split opening 12B. An annular finger-grip 14 is circumferentially formed at the end of the outer cylinder opening 4. The front chamber outer cylinder 1A and the rear chamber outer cylinder 1B are disposed in series oppositely to one another over the entire peripheries of the inner peripheral edge 19A of the split opening 12A and the inner peripheral edge 19B of the split opening 12B. The front chamber outer cylinder 1A and a rear chamber outer cylinder 1B are coupled by a coupling component 10A, 10B. The coupling component 10A, 10B is made of plastics and has elasticity and has a cylindrical shape.

A sealing plug 6A, 6B is split in a direction perpendicular to a thickness direction, and divided into a first sealing plug 6A and a second sealing plug 6B. The sealing plug 6A and the sealing plug 6B contact each other in such a manner that the sealing plug 6A is liquid-tightly positioned in a front chamber outer cylinder 1A near a split opening 12A and the sealing plug 6B is liquid-tightly positioned in a rear chamber outer cylinder 1B near a split opening B. A distance N from the end face of the sealing plug 6A near the end 5 to the end face of the sealing plug 6B near the outer cylinder opening 4 is set shorter than the length L of a bypass groove 3. The coupling component 10A, 10B is radially split, and divided into a coupling component 10A and a coupling component 10B. An annular flange A is circumferentially formed on the outer peripheral surface of one axial end of the coupling component 10A, and a recess 15A is circumferentially formed on the inner peripheral surface near the end with the flange A. An annular flange 40B is circumferentially formed on the outer periphery of the one axial end of the coupling component 10B, and a recess 15B is circumferentially formed on the inner peripheral surface near the end with the flange 40B. A protrusion 13A of the front chamber outer cylinder 1A is engaged with the recess 15A of the coupling component 10A, and the coupling portion 16A of the front chamber outer cylinder 1A is liquid-tightly held by the coupling component 10A. A protrusion 13B of the rear chamber outer cylinder 1B is engaged with the recess 40B of the coupling component 10B, and coupling portion 16B of the rear chamber outer cylinder 1B is liquid-tightly held by the coupling component 10B. The flange 40A of the coupling component 10A is liquid-tightly adhered oppositely to the flange 40B of the coupling component 10B.

Thus, the first inner chamber 8 is formed within the front chamber outer cylinder 1A between the sealing plug 6A, 6B and an end 5 closed with a sealing plug 20. A plunger 7 made of rubber is liquid-tightly sealed at a position of the sealing plug 6A, 6B near the outer cylinder opening 4 in the rear chamber outer cylinder 1B to form a second inner chamber 9 between the plunger 7 and the sealing plug 6B. Female threads 18 are formed on the plunger 7 from the end face of the outer cylinder opening 4 so as to mount a syringe inner cylinder 30.

The end 5 of the front chamber outer cylinder 1A is formed in a wide port bottle shape. A bottom 21 of a bottomed hollow sealing plug 20 made of rubber is liquid-tightly engaged with an inner surface of the end 5 facing the first inner chamber 8 so that the end 5 is liquid-tightly closed. A base cylinder 23 of a needle mounting cylinder 22 is sealed with the hollow section of the sealing plug 2a. The mounting cylinder 22 protrudes from the end opening 17 of the end 5. A cylindrical hole 25 is perforated through from the end face of a tip 24 to the end face of the base cylinder 23. The outer peripheral surface of the tip 24 is gradually reduced in diameter toward the end as a lure tapered surface 26. A guard base 28 of a bottomed hollow guard 27 made of aluminum is gas-tightly secured to the outer peripheral surface of the end 5 by caulking. The tip 24 of the needle mounting cylinder 22 is sealed in the hollow section of the guard 27. A collapsible brittle portion 29 is circumferentially formed in the guard 27 at a the largest diameter portion of the lure tapered surface 26 of the tip 24.

Since the end 5 of the front chamber outer cylinder 1A is constructed in the above-described structure, the tip 24 to be mounted with a syringe needle can be reliably held in the guard 27 in a sterilized state up to the time of injection by sterilizing such as heating to sterilize the front chamber outer cylinder 1A before powdered medicine is sealed in the front chamber outer cylinder 1A.

In order to seal the powdered medicine in a sterilized state in the front chamber outer cylinder 1A after the interior of the guard 27 is sterilised, a predetermined quantity of medicine liquid to be freeze-dried to powdered medicine is filled in the front chamber outer cylinder 1A, the front chamber outer cylinder 1A is set in a freeze-drying apparatus of sterilised state, the medicine liquid is freeze-dried, a sealing rubber plug 37 having a ventilation groove 38 is inserted into the split opening 12A by an insertion rod 36 set in the apparatus, and the split opening 12A is sealed by the sealing rubber plug 37 so that the powdered medicine is sealed in a sterilized state in the front chamber outer cylinder 1A.

As shown in Figure 15, in a conventional dual chamber prefillable syringe, since a syringe outer cylinder 1 is formed of one-piece, it is necessary to insert a sealing plug 1f substantially to the centre in the outer cylinder la by an inserting rod u and to seal powdered medicine in a sterilized state in the outer cylinder la. Thus, the length of the conventional dual chamber prefillable syringe is increased relating to the length of the rod u set in a freeze-drying apparatus. On the other hand, according to the dual chamber prefillable syringe as shown in Figure 16, since the syringe outer cylinder 1 is split into the front chamber outer cylinder 1A and the rear chamber outer cylinder 1B, it is possible to seal the powder medicine in a sterilized state in the front chamber outer cylinder 1A by sealing the split opening 12A in the front chamber outer cylinder 1A by the sealing rubber plug 37. Therefore, the length of the inserting rod 36 can be shortened as compared with that of the conventional inserting rod u, and the space in the freeze-drying apparatus occupied by the inserting rod 36 is reduced to increase the space set for setting the front chamber outer cylinder 1A. In addition, since the length of the front chamber outer cylinder 1A is shorter than that of the conventional syringe outer cylinder lA, the number of the front chamber outer cylinders 1A which can be set in the freeze-drying apparatus is much increased as compared with the conventional syringe outer cylinder 1A. Therefore, the production efficiency of the freeze-drying work by the freeze-drying apparatus can be improved.

In order to seal liquid medicine in a sterilized state in the rear chamber outer cylinder 1B, the coupling portion 16B of the rear chamber outer cylinder component 1B is engaged with the coupling component 10A, 10B with the recess and the protrusion, the sealing plug 6A, 6B is sealed in the rear chamber outer cylinder 1B near the split opening 12B, a predetermined quantity of the liquid medicine is filled through the outer cylinder opening 4 into the rear chamber outer cylinder 1B, and the plunger 7 is further sealed in the rear chamber outer cylinder 1B near the outer cylinder opening 4 to seal the liquid medicine between the sealing plug 6A, 6B and the plunger 7. Thereafter, the rear chamber outer cylinder 1B is sterilized by a thermal sterilization, etc., to sterilize the liquid medicine sealed in the rear chamber outer cylinder 1B.

The front chamber outer cylinder 1A in which the powdered medicine is sealed in a sterilized state and the rear chamber outer cylinder 1B in which the liquid medicine is sealed in a sterilized state are assembled by removing the sealing rubber plug 37 for sealing the split opening 12A of the front chamber outer cylinder 1A, contacting the rear chamber outer cylinder 1B at the coupling component 10 side with the coupling portion 16A of the front chamber outer cylinder 1A, coupling the front chamber outer cylinder 1A and the rear chamber outer cylinder 1B in series by the coupling component 10A, 10B, and pressing the plunger 7 to move the sealing plug 6A, 6B to the position where the front chamber outer cylinder 1A and the rear chamber outer cylinder 1B are opposed to one another.

According to the conventional dual chamber prefillable syringe as shown in Figure 14, when liquid medicine q is filled and sealed in a syringe outer cylinder 1A, since the outer cylinder 1A is formed of one piece, it is necessary to seal powdered medicine s in the outer cylinder 1A in a sterilized state and to then fill and seal liquid medicine q in a sterilized environment in the outer cylinder 1a including the powder medicine s. Therefore, as already described above, there is a danger that the liquid medicine q, the powdered medicine s and the interior of the syringe outer cylinder 1a are contaminated by the introduction of bacteria, etc., during filling and sealing procedures of the liquid medicine q even in the sterilized environment. On the other hand, according to the present invention, since the syringe outer cylinder 1 is split into the front chamber outer cylinder 1A and the rear chamber outer cylinder 1B, the step of sealing a predetermined quantity of the powdered medicine in a sterilized state in the front chamber outer cylinder 1A and the step of sealing a predetermined quantity of liquid medicine in a sterilized state in the rear chamber outer cylinder 1B can be effected simultaneously. Therefore, according to the present invention, it is not required after the powdered medicine is sealed in a sterilized state to fill and seal the liquid medicine in the syringe outer cylinder 1 including the powdered medicine, so that the danger of the liquid medicine, powdered medicine and the interior of the syringe outer cylinder 1 being contaminated by bacteria, etc., during these procedures is eliminated.

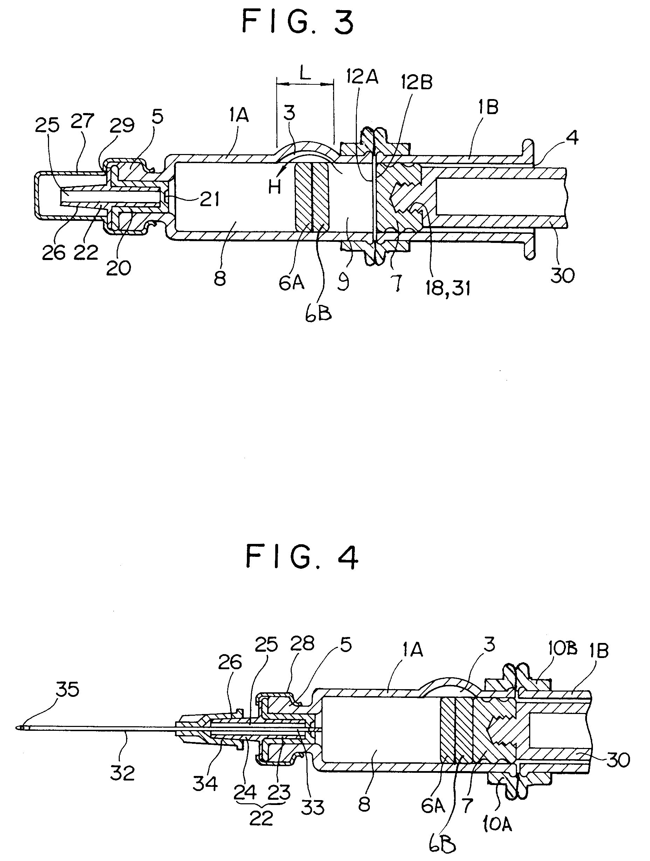

In the case of injection, male threads 31 of the syringe inner cylinder 30 are engaged with the female threads 18 of the plunger 7, and the inner cylinder 30 is mounted at the plunger 7, as shown in Figure 3. The plunger 7 is pressed forwardly in the outer cylinder 1 so that the hydraulic pressure of the liquid medicine is raised by pressing forward the plunger 7, and the sealing plug 6 is pressed forward to the bypass groove 3 side due to an increase in the hydraulic pressure of the liquid medicine. Thus, when the sealing plug 6A, 6B is introduced into a range of the length L of the bypass groove 3, the first inner chamber 8 communicates with the second inner chamber 9 through the bypass groove 3 so that the pressures in both the inner chambers 8 and 9 become uniform. Therefore, the sealing plug 6A, 6B is stopped, only the plunger 7 is then continuously pressed forward, the liquid medicine is, in turn, fed into the first inner chamber 8 as indicated by an arrow H, the plunger 7 contacts the sealing plug 6A, 6B thereby to feed the entire quantity of the liquid medicine into the first inner chamber 8. As a result, when the powdered medicine is mixed with the liquid medicine in the first inner chamber 8, a parenteral solution is produced. Thereafter, as shown in Figure 4, a side pressure is applied to the guard 27 by a finger head to break the guard 27 at the collapsible brittle portion 29 to expose the cylinder end 24. A short needle 33 of a double flank syringe needle 32 is introduced in the cylindrical hole 25 of the tip 24 and the needle base 34 of the double flank syringe needle 32 is mounted on the lure tapered surface 26 of the tip 24. The short needle 33 is pierced to the bottom 21 of the sealing plug 20 to communicate a needle hole 35 of the double flank syringe needle 32 with the first inner chamber 8.

It is noted that the first embodiment according to the present invention is not limited in shape and materials to the particular shape and materials described above. In the embodiment described above, only one bypass groove 3 is employed. However, this invention is not limited to this particular embodiment. For example the number of the bypass grooves 3 is not limited to only one, but may include a plurality of bypass grooves 3.

In order to seal powdered medicine in a sterilized state in the front chamber outer cylinder 1A, the front chamber outer cylinder 1A is sealed with the coupling component 10A with the engagement of the recess and protrusion, the front chamber outer cylinder 1A is then sterilized by a thermal sterilization, etc. to sterilize the interior of the guard 27. Then, a predetermined quantity of medicine liquid to be freeze-dried to powdered medicine is filled in the front chamber outer cylinder 1A, the outer cylinder 1A is set within a freeze-drying apparatus of sterilized state, and the medicine liquid is freeze-dried to powdered medicine. The sealing plug 6A is subsequently inserted through the split opening 12A by an inserting rod set in the freeze-drying apparatus therein, and the split opening 12A is sealed by the sealing plug 6A to seal the powdered medicine in a sterilized state in the front chamber outer cylinder 1A.

In order to seal the liquid medicine in a sterilized state in the rear chamber outer cylinder 1B, the rear chamber outer cylinder 1B is sealed with the coupling component 10B with the engagement of the recess and the protrusion, and the split opening 12B is sealed with the sealing plug 6B. A predetermined quantity of liquid medicine is filled through the outer cylinder opening 4 into the rear chamber outer cylinder 1B, the plunger 7 is sealed in the rear chamber outer cylinder 1B near the outer cylinder opening 4, and the liquid medicine is sealed in the rear chamber outer cylinder 1B between the sealing plug 6B and the plunger 7. Thereafter, the rear chamber outer cylinder 1B is sterilized by a heating sterilization, etc., to seal the liquid medicine in a sterilized state in the rear chamber outer cylinder 1B.

Then, the flange 40A of the coupling component 10A is liquid-tightly adhered to the flange 40B of the coupling component 10B in a sterilized environment the front chamber outer cylinder 1A in which a predetermined quantity of powdered medicine is sealed in a sterilized state and the rear chamber outer cylinder 1B in which a predetermined quantity of the liquid medicine is sealed in a sterilized state are coupled in series with one another.

According to this embodiment, the sealing state in the front chamber outer cylinder 1A is not opened during the assembling procedure. Therefore, the invasion of bacteria, etc. into the powdered medicine in the front chamber outer cylinder 1 is reliably prevented, and the danger of contaminating the powdered medicine by bacteria during the assembling work is much reduced.

The sealing plug 6A contacts the sealing plug 6B. However, it is noted that, if a distance N between the sealing plug 6A and the sealing plug 6B falls within a shorter range than the length L of the bypass groove 3, the sealing plug 6A and the sealing plug 6B may be separated. The method of coupling the split coupling component 10A to the coupling component 10B not limited to the above-described adhering method, but may be a method of thermal fusion-bonding.

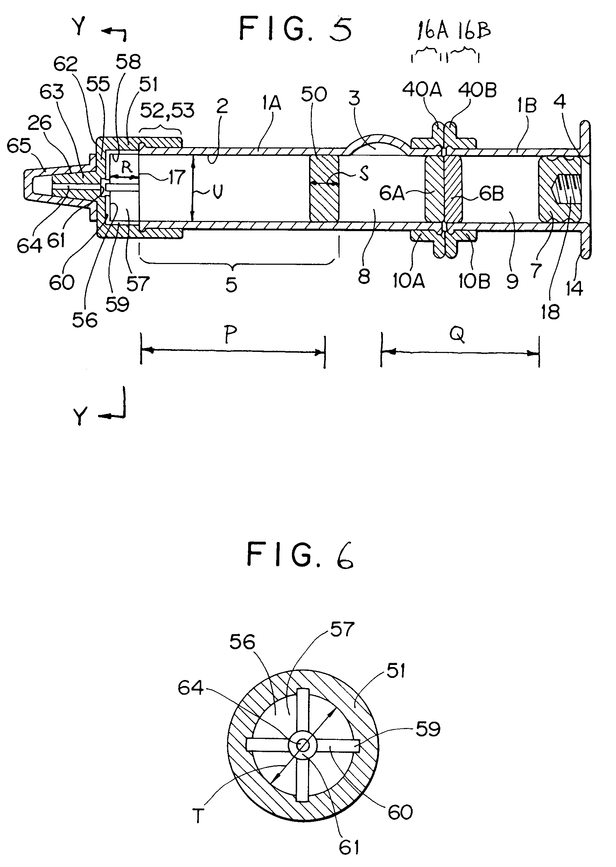

A second embodiment according to the present invention will be described with reference to Figures 5 to 8. In comparison with the first embodiment as shown in Figures 1 to 4, the same device and components are denoted by the same reference numerals and symbols, and only different structure, operation and effect will be described.

A cylindrical end 5 of a front chamber outer cylinder 1A formed in the same cylindrical shape as the coupling portion 16A of the outer cylinder 1A. A stopper 50 made of rubber is liquid-tightly sealed in the front chamber outer cylinder. A distance P from an end opening 17 to the stopper 50 is set longer than a distance Q from a bypass groove 3 to the end face of a plunger 7 at the side of the bypass groove 3. A bottomed cylindrical hollow end housing 51 is made of plastics with elasticity. An end 53 of the cylindrical end 5 is gas-tightly sealed in a coupling portion 52 of the housing 51. A columnar space from the end opening 17 of the end 5 to an inner surface 56 of a bottom 55 of the housing 51 is formed as a bypass chamber 57. The bypass chamber 57 has a length R set longer than the thickness S of the stopper 50. A diameter T of the bypass chamber 57 is the same as the inner diameter U of the end opening 17. The centre of the diameter T coincides with the centre of the inner diameter U. A sectional circular flat surface of the bypass chamber 57 contacts and coincides with the end opening 17. In the end housing 51, four bypasses 9 are recessed longitudinally on a housing inner wall 58 for forming the bypass chamber 57. A bottom groove 60 connected to the bypasses 59 is radially recessed on the surface 56 of the housing bottom 55. A housing recess 61 is formed at the centre of the inner surface 56 where all the bottom grooves 60 are concentrated so that all the bottom grooves 60 are connected to the housing recess 61. An inverted frustoconical needle mounting portion 63 extends from the outer surface 62 of the housing bottom 55. A small hole 64 is perforated through from a frustum face 66 to the housing recess 61 in the needle mounting portion 63. An outer peripheral surface of the needle mounting portion 63 is gradually reduced in diameter toward its end as a lure tapered surface 26. A bottomed hollow cap 65 made of plastics with elasticity is tapered in the same taper as the lure tapered surface 26 of the needle mounting portion 63 on the inner wall surface of the hollow section, and the needle mounting portion 63 is liquid-tightly sealed in the hollow section.

Since the second embodiment shown in Fig. 5 is formed in a structure of the cylindrical end 5 as described above, the needle mounting portion 63 can be held in a sterilized state by the cap 65 sterilizing, such as thermally, the front chamber outer cylinder 1A before powdered medicine is sealed in the outer cylinder 1A, and the interior of the small hole 64 of the needle mounting portion 63, the bypass chamber 57 and the interior of the cylindrical end 5 from the stopper 50 to the end opening 17 can be sealed in a sterilized state.

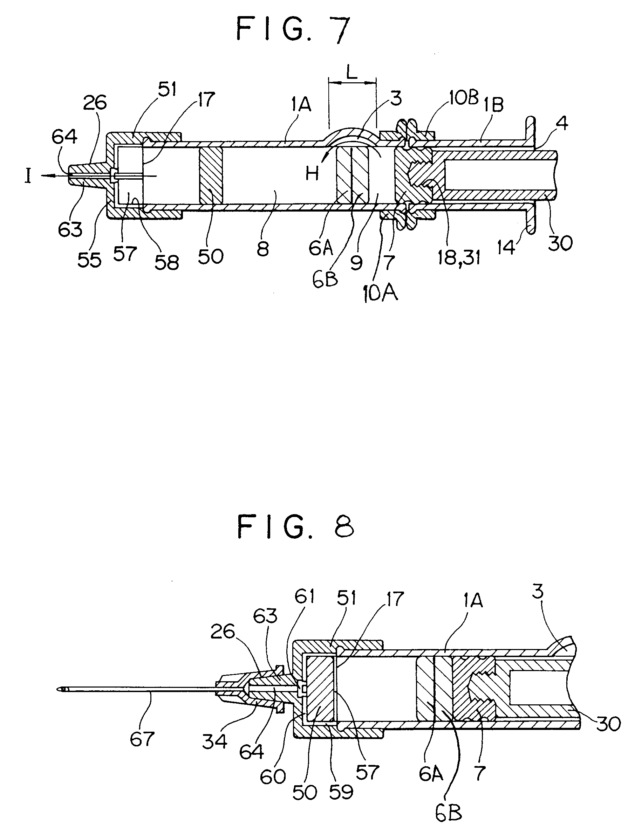

Powdered medicine is sealed in a sterilized state in a first inner chamber 8 between the stopper 50 and the sealing plug 6A, 6B, and liquid medicine is sealed in a sterilized state in a second inner chamber 9 between the sealing plug 6A, 6B and the plunger 7. In the case of injection, as shown in Figure 7, the cap 65 is removed, and the plunger 7 is pressed forward by the syringe inner cylinder 30. Thus, the hydraulic pressure of the liquid medicine is raised by pressing the plunger 7, and the sealing plug 6A, 6B is pressed forward by the raised hydraulic pressure of the medicine. The air in the second inner chamber 9 is compressed by pressing the sealing plug 6A, 6B to raise pneumatic pressure in the second inner chamber 9 so that the stopper 50 is pressed forward by the raised pneumatic pressure. Since the cap 65 is removed, the bypass chamber 57 communicates with the atmosphere. Therefore, even if the stopper 50 is pressed forward, the air in the cylindrical end 5 is escaped into the atmosphere as indicated by an arrow I in Figure 7, and hence the internal pressure of the bypass chamber 57 is not raised. Therefore, the sealing plug 6A, 6B is smoothly pressed forward to the bypass groove 3 side. When the sealing plug 6A, 6B is moved to a range of the length L of the bypass groove 3, the first inner chamber 8 communicates with the second inner chamber 9 through the bypass groove 3. Then, the sealing plug 6A, 6B is stopped, only the plunger 7 is thereafter continuously pressed forward to feed the liquid medicine into the first inner chamber 8. When the plunger 7 reaches to the sealing plug 6A, 6B, the entire quantity of the liquid medicine is fed into the first inner chamber 8, and the powdered medicine and the liquid medicine are mixed in the first inner chamber 8 to produce a parenteral solution.

The stopper 50 is also pressed forward by pressing the plunger 7. Since a distance P from the end opening 17 of the cylindrical end 5 to the stopper 50 is set longer than a distance Q from the bypass groove 3 to the end face of the plunger 7 at the bypass groove 3 side, the stopper 50 is not pressed forward into the interior of the bypass chamber 57 until the entire quantity of the liquid medicine is fed to the first inner chamber 8. After the parenteral solution is produced, when the plunger 7 is further pressed forwards the stopper 50 is pressed forward into the chamber 57. When the stopper 50 is introduced into the bypass chamber 57, the stopper 50 contacts the housing bottom 55 of the end housing 51. However, the parenteral solution is not blocked by the stopper 50, is fed from the bypasses 59 of the housing inner wall 58 through the bottom groove 60 to the housing recess 61, is further fed to the hole 64 of the needle mounting portion 63 perforated to the housing recess 61, and is expelled from the dual chamber prefillable syringe. When the sealing plug 6 is contacted with the stopper 50, the entire quantity of the parenteral solution is exhausted.

The needle base 34 of the syringe needle 67 is mounted on the lure tapered surface 26 of the needle mounting portion 63, and the syringe needle 67 is mounted at the needle mounting portion 63. The syringe 67 is not constricted in a structure in which the bottom 21 of the sealing plug 20 is pierced by the short needle 33 of the double flank syringe needle 32 as in the embodiment shown in Figure 4.

In the second embodiment shown in Figures 5 to 8, its shape and materials are not limited to the above-described shape and materials.

The structure, operation and effect of the embodiment shown in Figures 5 to 8 are all included in the further embodiment shown in Figures 1 to 4.

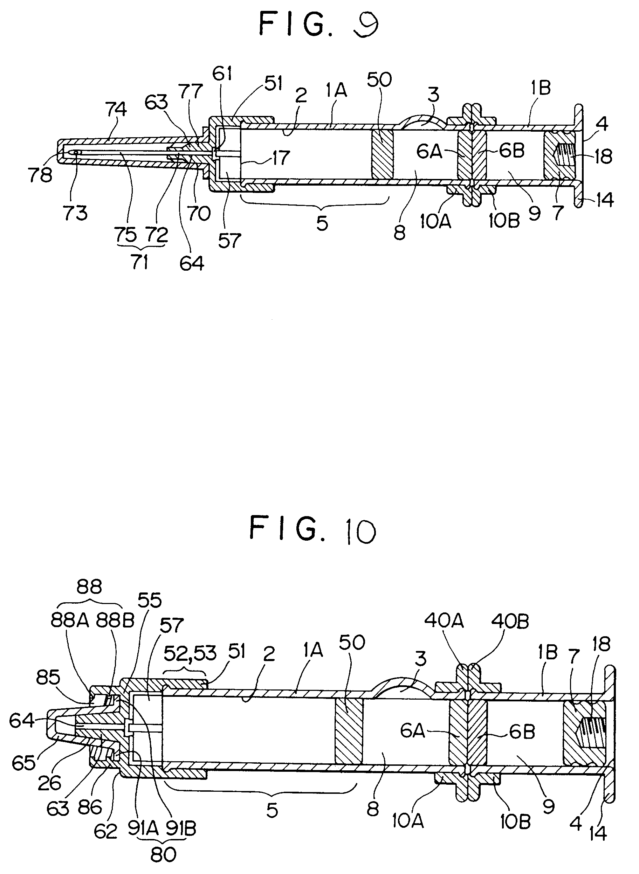

A third embodiment according to the present invention is shown in Figure 9. The structure, operation and effect of the embodiment shown in Figure 9 are all included in the first embodiment shown in Figures 1 to 4. The reference numerals and symbols of the further embodiment shown in Figure 9 are the same as those of the embodiment shown in Figures 1 to 4.

A fourth embodiment according to the present invention is shown in Figure 10. The structure, operation and effect of the embodiment shown in Figure 10 are all included in the first embodiment shown in Figures 1 to 4. The reference numerals and symbols of the embodiment shown in Figure 10 are the same as those of the embodiments shown in Figures 1 to 4.

In all the embodiments of the above-described assembling type dual chamber prefillable syringe as described above, the descriptions have been made in such a manner that a predetermined quantity of the powdered medicine freeze-dried by the freeze-drying apparatus is sealed in a sterilized state in the first inner chamber 8 and a predetermined quantity of the liquid medicine of dissolved solution for dissolving the powdered medicine is sealed in a sterilized state in the second inner chamber 9. However, the medicine sealed in first inner chamber 8 is not limited to the powdered medicine freeze-dried by the freeze-drying apparatus, and the medicine sealed in the second inner chamber 9 is not limited to the liquid medicine of dissolved solution for dissolving the powdered medicine. In other words, in the assembling type dual chamber prefillable syringe according to this invention, a predetermined quantity of powdered medicine or liquid medicine can be sealed in a sterilized state in the first inner chamber 8 and a predetermined quantity of liquid medicine can be sealed in a sterilized state in the second inner chamber 9. However, the effect of this invention is particularly performed in the case that a predetermined quantity of the powdered medicine freeze-dried by the freeze-drying apparatus is sealed in a sterilized state in the first inner chamber 8 and a predetermined quantity of the liquid medicine of dissolved solution for dissolving the powdered medicine is sealed in a sterilized state in the second inner chamber 9.

Since this invention is embodied in the structure and operated as described above, the following advantages can be provided.
(a) When the powdered medicine is sealed in a sterilized state in the first inner chamber in the syringe outer cylinder and the liquid medicine is sealed in a sterilized state in the second inner chamber, the liquid medicine is fed into the first inner chamber without opening the sealing states of the first and second inner chambers, the powdered and liquid medicines are mixed in the first inner chamber to produce the parenteral solution. Therefore, there is no danger of the parenteral solution being contaminated by the bacteria, etc., at the time of producing the parenteral solution.
(b) When the dual chamber prefillable syringe in which the powdered medicine is sealed in a sterilized state in the first inner chamber and the liquid medicine is sealed in a sterilized state in the second inner chamber is assembled, the step of sealing the powdered medicine in a sterilized state in the front chamber outer cylinder and the step of sealing the liquid medicine in a sterilized state in the rear chamber outer cylinder can be treated in parallel. Therefore, the filling and sealing steps of the liquid medicine in the syringe outer cylinder in which the powdered medicine is sealed in a sterilized state after the powdered medicine is sealed in a sterilized state like the conventional dual chamber prefillable syringe are eliminated, and there is no danger of the liquid medicine, powdered medicine and the interior of the syringe outer cylinder being contaminated by the mixture with bacteria, etc.
(c) Further, if the powdered medicine is sealed in a sterilized state in the front chamber outer cylinder, the production efficiency of the freeze-drying procedure by the freeze-drying apparatus is improved.
(d) The first inner chamber can reliably communicate with the second inner chamber through the bypass groove, and the assembling is facilitated.
(e) When the dual chamber prefillable syringe in which the powdered medicine is sealed in a sterilized state in the first inner chamber and the liquid medicine is sealed in a sterilized state in the second inner chamber is assembled, the first and second inner chambers need not be opened. Therefore, there is no danger of the powdered medicine and the liquid medicine being contaminated by the ingress of bacteria, etc.
(f) The cylindrical end of the needle mounting portion can be reliably held in a sterilized state in the guard up to the time of injection, and in the case of injection, the guard is broken at the collapsible brittle portion to expose the head, and the double flank syringe needle can be mounted at the head.
(g) The needle mounting portion, the interior of the small hole of the needle mounting portion, the bypass chamber 57 and the interior of the cylindrical end from the stopper to the end opening can be held in sterilized state.
(h) When the cap is removed in the case that the powdered medicine is sealed in a sterilized state in the first inner chamber in the syringe outer cylinder and the liquid medicine is sealed in a sterilized state in the second inner chamber, the liquid medicine is fed to the first inner chamber through the bypass groove, and the powdered medicine and the liquid medicine are mixed in the first inner chamber to produce a parenteral solution smoothly.
(i) When the syringe needle is mounted at the needle mounting portion, the sealing plug is not collapsed by the syringe needle. Therefore, the needle hole of the syringe needle is not blocked by the collapsed pieces, and the pieces are not introduced in vivo into the patient together with the parenteral solution.
(j) Mounting of the syringe needle on the needle mounting portion can be made easy.
(k) Labour involved in mounting the syringe needle on the needle mounting portion can be eliminated.
(l) The end needle of the needle tube, the needle mounting portion, the interior of the needle hole of the needle tube, the bypass chamber and the interior of the cylindrical end from the stopper to the end opening can be held in a sterilized state by the needle guard.
(m) Further, the end needle can be protected by the needle guard to prevent deformation of the end needle end, and the danger of imparting pain to a patient due to the deformation of the end needle end can be obviated.
(n) There may be no danger of the cap being removed from the needle mounting portion up to the time of injection. Therefore, the needle mounting portion, the interior of the small hole of the needle mounting portion, the bypass chamber and the interior of the cylindrical end from the stopper to the end opening is reliably held in a sterilized state by the cap up to the time of injection.

## Claims

1. A dual chamber prefillable syringe comprising,
a syringe outer cylinder (1) having a bypass groove (3) recessed longitudinally thereof on the inner wall (2) substantially at the centre thereof,
a sealing plug (6A,6B) of thickness (N) shorter than the length (L) of the bypass groove (3) and liquid-tightly sealed at a position near an outer cylinder opening (4) from the bypass groove (3) to form a first inner chamber (8) between a liquid-tightly sealed cylindrical end (5) thereof and the sealing plug (6A,6B),
a plunger (7) liquid-tightly sealed at a position near the outer cylinder opening (4) from the sealing plug (6A,6B) to form a second inner chamber (9) between the plunger (7) and the sealing plug (6A,6B),
the syringe outer cylinder (1) being split in a direction perpendicular to a longitudinal direction at a position near the outer cylinder opening (4) from the bypass groove (3) to be divided into a front chamber outer cylinder (1A) and a rear chamber outer cylinder (1B),
the front chamber outer cylinder (1A) including the bypass groove (3), the cylindrical end (5) and a first split opening (12A),
the rear chamber outer cylinder (1B) including the outer cylinder opening (4) and a second split opening (12B),
an inner peripheral edge of the split opening (12A) being opposed over an entire periphery to an inner peripheral edge of the second split opening (12B), so that the front chamber outer cylinder (1A) and the rear chamber outer cylinder (1B) are disposed in series, and
the front chamber outer cylinder (1A) and the rear chamber outer cylinder (1B) being liquid-tightly held by a coupling component (10), characterised in that
the sealing plug (6A,6B) comprises a first sealing plug (6A) and a second sealing plug (6B) by splitting the sealing plug (6A,6B) in a direction perpendicular to a thickness direction,
the first sealing plug (6A) is liquid-tightly sealed at a position near the first split opening (12A) in the front chamber outer cylinder (1A),
the second sealing plug (6B) is liquid-tightly sealed at a position near the second split opening (12B) of the rear chamber outer cylinder (1B), and
a distance from an end face of the first sealing plug (6A) at the bypass groove (3) side to an end face of the second sealing plug (6B) at the outer cylinder opening (4) side is set shorter than the length (L) of the bypass groove (3).

2. A dual chamber prefillable syringe according to claim 1,
characterised in that
the dual chamber prefillable syringe further comprises a needle mounting cylinder (22) including a base cylinder (23) and an end cylinder (24), a bottomed hollow guard (27), and a bottomed hollow sealing plug (20) made of an elastic material,
the cylindrical end (5) of the front chamber outer cylinder (1A) is formed in a wide port bottle shape,
the bottomed hollow sealing plug (20) is sealed in the cylindrical end (5) in such a manner that a bottom (21) of the bottomed hollow sealing plug (20) is faced towards the first inner chamber (8), so that the cylindrical end (5) is liquid-tightly sealed,
the base cylinder (23) of the needle mounting cylinder (22) is sealed in a hollow section of the sealing plug (20),
the end cylinder (24) of the needle mounting cylinder (22) protrudes from an end opening of the cylindrical end (5),
an outer peripheral surface of the end cylinder (24) is gradually reduced in diameter toward its end as a tapered surface (26),
a cylindrical hole (25) is perforated through the needle mounting cylinder (22) from an end face of the end cylinder (24) to an end face of the base cylinder (23),
a guard base (28) of the bottomed hollow guard (27) is gas-tightly secured to an outer wall of the cylindrical end (5) so that the end cylinder (24) of the needle mounting cylinder (22) is sealed in the guard (27), and
a collapsible brittle portion (29) is circumferentially formed at a position of widest diameter portion of the tapered surface (26).

3. A dual chamber prefillable syringe according to claim 1 or claim 2,
characterised in that
the dual chamber prefillable syringe further comprises a stopper (50) made of an elastic material, a bottomed hollow end housing (51) and a bottomed hollow cap (65) having an elasticity,
the stopper (50) is sealed in the cylindrical end (5) of the front chamber outer cylinder (1A) to liquid-tightly seal the cylindrical end (5),
a distance (P) from the end opening (17) of the cylindrical end (5) to the stopper (50) is set longer than a distance (Q) from the bypass groove (3) to an end face of the plunger (7) at the bypass groove (3) side,
an end (53) of the cylindrical end (5) is gas-tightly engaged within the coupling portion of the bottomed hollow end housing (51), so that a hollow section of the end housing (51) from the end opening (17) of the cylindrical end (5) to an inner surface of a bottom (55) of the end housing (51) is formed as a bypass chamber (57),
a length (R) of the bypass chamber (57) is set longer than a thickness (S) of the stopper (50),
the end opening (17) is included in a surface which contacts the bypass chamber (57),
a plurality of bypasses (59) are recessed longitudinally of the end housing (51) on an inner wall of the end housing (51),
a plurality of bottom grooves (60) coupled to the bypass (59) is recessed radially on an inner surface of the bottom (55) of the end housing (51),
a housing recess (61) is formed at a portion concentrated with all bottom grooves (60) to connect all the bottom grooves (60) to the housing recess (61),
a needle mounting portion (63) is suspended from an outer surface of the bottom (55) of the housing (51),
a small hole (64) from an end (66) of the needle mounting (63) to the housing recess (61) is perforated through the needle mounting portion (63), and
the needle mounting portion (63) is liquid-tightly sealed with the hollow section of the bottomed hollow cap (65).

4. A dual chamber prefillable syringe according to claim 3,
characterised in that
an outer wall surface of the needle mounting portion (63) is gradually reduced in diameter toward its end as a tapered surface (26), and
an inner wall surface of the bottomed hollow cap (65) is formed in the same tapered surface as the tapered surface (26).

5. A dual chamber prefillable syringe according to claim 3,
characterised in that
the dual chamber prefillable syringe further comprises a needle tube (71) and a cap (65, 74),
the outer wall surface of the needle mounting portion (63) is gradually reduced in diameter toward its end in a tapered surface,
a base needle (72) of the needle tube (71) is perforated through from the end of the small hole (64) of the needle mounting portion (63) to the housing recess (61), so that the base needle (72) is gas-tightly secured to the small hole (64) of the needle mounting portion (63),
a needle hole (73) perforated through the needle tube (71) is connected to the housing recess (61),
an end needle (75) of the needle tube (71) is provided to protrude from the needle mounting portion (63),
the cap (65, 74) acts as a needle guard,
a length (K) of a hollow section of the needle guard (74) is set longer than a total length (J) of the length of the end needle (75) and the length of the needle mounting portion (63),
an inner wall of the needle guard (65, 74) is formed in the same tapered surface as the tapered surface of the needle mounting portion (63), and
the end needle (75) is sealed in the hollow section of the needle guard (65, 74).

6. A dual chamber prefillable syringe according to claim 4, characterised in that
the dual chamber prefillable syringe further comprises a lock (86) having a columnar portion (85),
the lock (86) is suspended from the outer surface (62) of the housing bottom (55),
the columnar portion (85) includes the needle mounting portion (63) therein,
female threads (88) are formed on an inner peripheral surface of the columnar portion (85),
a flange (80) is formed on an outer wall surface of the cap (65), and
the flange (80) is engaged by the female thread (88) with the lock (86).

## Patentansprüche

1. Vorgefüllte Zweikammer-Spritze mit
- einem äußeren Spritzenzylinder (1) mit einer Umgehungsleitungsnut (3), die daran in Längsrichtung an der Innenwand (2) im wesentlichen an ihrem Zentrum ausgenommen ist,
- einem Dichtungsstöpsel (6A, 6B), der eine Dicke (N) aufweist, die kürzer als die Länge (L) der Umgehungsleitungsnut (3) ist, und der, von der Umgehungsleitungsnut (3) aus, an einer Position nahe einer Außenzylinderöffnung (4) flüssigkeitsdicht abgedichtet ist, um eine erste Innenkammer (8) zwischen einem flüssigkeitsdicht abgedichteten zylindrischen Ende (5) davon und dem Dichtungsstöpsel (6A, 6B) zu bilden,
- einem Kolben (7), der vom Dichtungsstöpsel (6A, 6B) aus an einer Position nahe der Außenzylinderöffnung (4) flüssigkeitsdicht abgedichtet ist, um eine zweite Innenkammer (9) zwischen dem Kolben (7) und dem Dichtungsstöpsel (6A, 6B) zu bilden,
- wobei der Spritzenaußenzylinder (1) von der Umgehungsleitungsnut (3) aus an einer Position nahe der Außenzylinderöffnung (4) in einer Richtung senkrecht zu einer Längsrichtung aufgespalten ist, um in einen Vorderkammer-Außenzylinder (1A) und einen Rückkammer-Außenzylinder (1B) unterteilt zu sein,
- wobei der Vorderkammer-Außenzylinder (1A) die Umgehungsleitungsnut (3), das zylindrische Ende (5) und eine erste Spaltöffnung (12A) umfaßt,
- wobei der Rückkammer-Außenzylinder (1B) die Außenzylinderöffnung (4) und eine zweite Spaltöffnung (12B) umfaßt,
- wobei eine innere Umfangskante der Spaltöffnung (12A) über einen gesamten Umfang einer inneren Umfangskante der zweiten Spaltöffnung (12B) gegenüberliegt, so daß der Vorderkammer-Außenzylinder (1A) und der Rückkammer-Außenzylinder (1B) in Reihe angeordnet sind, und
- wobei der Vorderkammer-Außenzylinder (1A) und der Rückkammer-Außenzylinder (1B) flüssigkeitsdicht durch eine Kupplungskomponente (10) gehalten werden,
**dadurch gekennzeichnet, daß**
- der Dichtungsstöpsel (6A, 6B) einen ersten Dichtungsstöpsel (6A) und einen zweiten Dichtungsstöpsel (6B) umfaßt, indem der Dichtungsstöpsel (6A, 6B) in einer Richtung senkrecht zu einer Dickenrichtung gespalten ist,
- der erste Dichtungsstöpsel (6A) im Vorderkammer-Außenzylinder (1A) an einer Position nahe der ersten Spaltöffnung (12A) flüssigkeitsdicht abgedichtet ist,
- der zweite Dichtungsstöpsel (6B) an einer Position nahe der zweiten Spaltöffnung (12B) des Rückkammer-Außenzylinders (1B) flüssigkeitsdicht abgedichtet ist, und
- ein Abstand von einer Stirnfläche des ersten Dichtungsstöpsels (6A) auf der Seite der Umgehungsleitungsnut (3) zu einer Stirnfläche des zweiten Dichtungsstöpsels (6B) auf der Seite der Außenzylinderöffnung (4) kürzer ausgebildet ist, als die Länge (L) der Umgehungsleitungsnut (3).

2. Vorgefüllte Zweikammer-Spritze nach Anspruch 1, **dadurch gekennzeichnet, daß**
- die vorgefüllte Zweikammer-Spritze ferner einen Nadelbefestigungszylinder mit einem Grundzylinder (23) und einem Endzylinder (24) umfaßt, einen hohlen, mit einem Boden versehenen Schutz (27) sowie einen hohlen, mit einem Boden versehenen Dichtungsstöpsel (20) aus einem elastischen Werkstoff,
- wobei das zylindrische Ende (5) des Vorderkammer-Außenzylinders (1A) in einer Flaschengestalt mit einer weiten Öffnung ausgebildet ist,
- wobei der hohle, mit einem Boden versehene Dichtungsstöpsel (20) im zylindrischen Ende (5) derart abgedichtet ist, daß ein Boden (21) des hohlen, mit einem Boden versehenen Dichtungsstöpsels (20) der ersten Innenkammer (8) zugewandt ist, so daß das zylindrische Ende (5) flüssigkeitsdicht abgedichtet ist,
- wobei der Grundzylinder (23) des Nadel-Befestigungszylinders (22) in einem hohlen Abschnitt des Dichtungsstöpsels (20) abgedichtet ist,
- wobei der Endzylinder (24) des Nadel-Befestigungs-Zylinders (22) von einer Endöffnung des zylindrischen Endes (5) hervorragt,
- wobei eine äußere Umfangsoberfläche des Endzylinders (24) als eine sich verjüngende Oberfläche (26) sich im Durchmesser zu ihrem Ende hin allmählich verringert,
- wobei ein zylindrisches Loch (25) durch den Nadel-Befestigungszylinder (22) von einer Stirnfläche des Endzyinders (24) zu einer Stirnfläche des Grundzylinders (23) perforiert ist,
- wobei eine Schutzbasis (28) des hohlen, mit einem Boden versehenen Schutzes (27) gasdicht an einer Außenwand des zylindrischen Endes (5) befestigt ist, so daß der Endzylinder (24) des Nadel-Befestigungszylinders (22) im Schutz (27) abgedichtet ist, und
- wobei ein faltbarer spröder Abschnitt (29) am Umfang an einer Position des Abschnitts mit dem größten Durchmesser der sich verjüngenden Oberfläche (26) ausgebildet ist.

3. Vorgefüllte Zweikammer-Spritze nach Anspruch 1 oder 2, **dadurch gekennzeichnet, daß**
- die vorgefüllte Zweikammer-Spritze ferner einen Stopfen (50) aus einem elastischen Werkstoff umfaßt, ein hohles, mit einem Boden versehenes Endgehäuse (51) und eine hohle, mit einem Boden versehene Kappe (65), die eine Elastizität aufweist,
- wobei der Stopfen (50) im zylindrischen Ende (5) des Vorderkammer-Außenzylinders (1A) abgedichtet ist, um das zylindrische Ende (5) flüssigkeitsdicht abzudichten,
- wobei ein Abstand (P) von der Endöffnung (17) des zylindrischen Endes (5) zum Stopfen (50) länger ausgebildet ist, als ein Abstand (Q) von der Umgehungsleitungnut (3) zu einer Stirnfläche des Kolbens (7) auf der Seite der Umgehungsleitungsnut (3),
- wobei ein Ende (53) des zylindrischen Endes (5) gasdicht mit dem Kupplungsabschnitt des hohlen, mit einem Boden versehenen Endgehäuses (51) in Eingriff steht, so daß ein hohler Abschnitt des Endgehäuses (51) von der Endöffnung (17) des zylindrischen Endes (5) bis zu einer Innenoberfläche eines Bodens (55) des Endgehäuses (51) als eine Umgehungskammer (57) ausgebildet ist,
- wobei eine Länge (R) der Umgehungskammer (57) länger ausgebildet ist als eine Dicke (S) des Stopfens (50),
- wobei die Endöffnung (17) in einer Oberfläche eingeschlossen ist, die mit der Umgehungskammer (57) in Berührung steht,
- wobei eine Vielzahl von Umgehungsleitungen (59) in Längsrichtung des Endgehäuses (51) an einer Innenwand des Endgehäuses (51) ausgenommen sind,
- wobei eine Vielzahl von Bodennuten (60), die mit der Umgehungsleitung (59) gekoppelt sind, an einer Innenoberfläche des Bodens (55) des Endgehäuses (51) radial ausgenommen ist,
- wobei eine Gehäuseausnehmung (61) an einem Abschnitt, an dem sich alle Bodennuten (60) konzentrieren, ausgebildet ist, um alle Bodennuten (60) mit der Gehäuseausnehmung (61) zu verbinden,
- wobei ein Nadel-Befestigungsabschnitt (63) an einer Außenoberfläche des Bodens (55) des Gehäuses (51) angehängt ist,
- wobei ein kleines Loch (64) von einem Ende (66) der Nadelbefestigung (63) durch den Nadel-Befestigungsabschnitt (63) zur Gehäuseausnehmung (61) perforiert ist, und
- wobei der Nadel-Befestigungsabschnitt (63) flüssigkeitsdicht mit dem hohlen Abschnitt der hohlen, mit einem Boden versehenen Kappe (65) abgedichtet ist.

4. Vorgefüllte Zweikammer-Spritze nach Anspruch 3, **dadurch gekennzeichnet, daß**
- eine Außenwandoberfläche des Nadel-Befestigungsabschnittes (63) als eine sich verjüngende Oberfläche (26) sich im Durchmesser zu ihrem Ende hin allmählich verringert, und
- eine Innenwandoberfläche der hohlen, mit einem Boden versehenen Kappe (65) in der gleichen, sich verjüngenden Oberfläche wie die sich verjüngende Oberfläche (26) ausgebildet ist.

5. Vorgefüllte Zweikammer-Spritze nach Anspruch 3, **dadurch gekennzeichnet, daß**
- die vorgefüllte Zweikammer-Spritze ferner ein Nadelrohr (71) und eine Kappe (65, 74) umfaßt,
- wobei die Außenwandoberfläche des Nadel-Befestigungsabschnitts (63) sich als sich verjüngende Oberfläche im Durchmesser zu ihrem Ende hin allmählich verringert,
- wobei eine Grundnadel (72) des Nadelrohrs (71) vom Ende des kleinen Lochs (64) des Nadel-Befestigungsabschnitts (63) zur Gehäuseausnehmung (61) hindurchperforiert ist, so daß die Grundnadel (72) gasdicht am kleinen Loch (64) des Nadel-Befestigungsabschnitts (63) befestigt ist,
- wobei ein Nadelloch (73), das durch das Nadelrohr (71) hindurchperforiert ist, mit der Gehäuseausnehmung (61) verbunden ist,
- wobei eine Endnadel (75) des Nadelrohrs (71) vorgesehen ist, die vom Nadel-Befestigungsabschnitt (63) hervorragt,
- wobei die Kappe (65, 74) als ein Nadelschutz wirkt,
- wobei eine Länge (K) eines hohlen Abschnitts des Nadelschutzes (74) länger ausgebildet ist als eine Gesamtlänge (J) der Länge der Endnadel (75) und der Länge des Nadel-Befestigungsabschnittes (65),
- wobei eine Innenwand des Nadelschutzes (65, 74) mit der gleichen, sich verjüngenden Oberfläche wie die sich verjüngende Oberfläche des Nadel-Befestigungsabschnitts (63) ausgebildet ist, und
- wobei die Endnadel (75) im hohlen Abschnitt des Nadelschutzes (65, 74) abgedichtet ist.

6. Vorgefüllte Zweikammer-Spritze nach Anspruch 4, **dadurch gekennzeichnet, daß**
- die vorgefüllte Zweikammer-Spritze ferner eine Sperre (68) mit einem Säulenabschnitt (85) umfaßt,
- wobei die Sperre (68) an die Außenoberfläche (62) des Gehäusebodens (55) angehängt ist,
- wobei der Säulenabschnitt (85) darin den Nadel-Befestigungsabschnitt (63) umfaßt,
- wobei weibliche Gewindegänge (88) an einer inneren Umfangsoberfläche des Säulenabschnitts (85) ausgebildet sind,
- wobei ein Flansch (80) an einer Außenwandoberfläche der Kappe (65) ausgebildet ist, und
- wobei der Flansch (80) vom weiblichen Gewindegang (88) mit der Sperre (86) eingegriffen wird.

## Revendications

1. Seringue à chambre double, qui peut être préalablement emplie et qui comprend
un cylindre extérieur (1) de seringue comportant une gorge de dérivation (3) formant un chambrage dans la direction de sa longueur sur la paroi intérieure (2), sensiblement en son centre,
un bouchon obturateur (6A, 6B) d'une épaisseur (N) plus faible que la longueur (L) de la gorge de dérivation (3) et monté de manière étanche aux liquides en un emplacement proche d'un orifice (4) du cylindre extérieur, à distance de la gorge de dérivation (3) de manière à former une première chambre interne (8) entre une extrémité cylindrique (5) de cette dernière, qui est obturée de manière étanche aux liquides, et le bouchon obturateur (6A, 6B),
un piston plongeur (7) monté de manière étanche aux liquides en un emplacement proche de l'orifice (4) du cylindre extérieur, à distance du bouchon obturateur (6A, 6B), de manière à former une seconde chambre interne (9) entre le piston plongeur (7) et le bouchon obturateur (6A, 6B),
le cylindre extérieur (1) de la seringue étant subdivisé dans une direction perpendiculaire à une direction longitudinale, en un emplacement proche de l'orifice (4) du cylindre extérieur, à distance de la gorge de dérivation (3), de manière qu'il soit divisé en un cylindre extérieur (1A) de chambre antérieure et un cylindre extérieur (1B) de chambre arrière,
le cylindre extérieur (1A) de la chambre antérieure comprenant la gorge de dérivation (3), l'extrémité cylindrique (5) et un premier orifice (12A) de la subdivision,
le cylindre extérieur (1B) de la chambre arrière comprenant l'orifice (4) du cylindre extérieur et un second orifice (12B) de la subdivision,
un bord périphérique intérieur de l'orifice (12A) de la subdivision étant placé, sur une périphérie totale, en face d'un bord périphérique interne du second orifice (12B) de la subdivision de manière que le cylindre extérieur (1A) de la chambre antérieure et le cylindre extérieur (1B) de la chambre arrière soient montés en série et
le cylindre extérieur (1A) de la chambre antérieure et le cylindre extérieur (1B) de la chambre arrière étant tenus de manière étanche aux liquides par un composant d'assemblage (10), caractérisée en ce que
le bouchon obturateur (6A, 6B) comprend un premier bouchon obturateur (6A) et un second bouchon obturateur (6B) par subdivision du bouchon obturateur (6A, 6B) dans une direction perpendiculaire à une direction de son épaisseur,
le premier bouchon obturateur (6A) est monté de manière étanche aux liquides en un emplacement proche du premier orifice (12A) de la subdivision dans le cylindre extérieur (1A) de la chambre antérieure,
le second bouchon obturateur (6B) est monté de manière étanche aux liquides en un emplacement proche du second orifice (12B) résultant de la subdivision et faisant partie du cylindre extérieur (1B) de la chambre arrière, et
une distance comprise entre une surface d'extrémité du premier bouchon obturateur (6A) située sur le côté de la gorge de dérivation (3) et une surface d'extrémité du second bouchon obturateur (6B) située sur le côté de l'orifice (4) du cylindre extérieur est calculée de manière qu'elle soit plus courte que la longueur (L) de la gorge de dérivation (3).

2. Seringue à chambre double pouvant être préalablement emplie selon la revendication 1,
caractérisée en ce que la seringue à chambre double pouvant être préalablement emplie comprend par ailleurs un cylindre (22) de montage d'une aiguille qui comprend un cylindre de base (23) et un cylindre d'extrémité (24), un élément creux de protection (27) comportant un fond et un bouchon obturateur creux (20) comportant un fond et réalisé en une matière élastique,
l'extrémité cylindrique (5) du cylindre extérieur (1A) de la chambre antérieure présente une forme de bouteille à orifice large,
le bouchon obturateur creux (20) comportant un fond est logé de manière étanche dans l'extrémité cylindrique (5) de manière qu'un fond (21) du bouchon obturateur creux (20) comportant un fond soit tourné vers la première chambre interne (8), de manière que l'extrémité cylindrique (5) soit obturée de manière étanche aux liquides,
le cylindre de base (23) du cylindre (22) de montage de l'aiguille est logé de manière étanche dans une partie creuse du bouchon obturateur (20),
le cylindre d'extrémité (24) du cylindre (22) de montage de l'aiguille est saillant sur un orifice d'extrémité de l'extrémité cylindrique (5),
une surface périphérique extérieure du cylindre d'extrémité (24) a un diamètre qui diminue progressivement vers son extrémité et qui forme une surface conique (26),
un trou cylindrique (25) passe dans le cylindre (22) de montage de l'aiguille entre une surface d'extrémité du cylindre d'extrémité (24) et une surface d'extrémité du cylindre de base (23),
une embase de protection (28) de l'élément de protection creux (27) comportant un fond est fixée de manière hermétique aux gaz sur une paroi extérieure de l'extrémité cylindrique (5) de manière que le cylindre d'extrémité (24) du cylindre (22) de montage de l'aiguille soit isolé par l'élément de protection (27) et
une partie cassante écrasable (29) est réalisée à la circonférence en un emplacement de la partie du plus grand diamètre de la surface conique (26).

3. Seringue à chambre double pouvant être préalablement emplie selon la revendication 1 ou la revendication 2,
caractérisée en ce que la seringue à chambre double pouvant être préalablement emplie comprend par ailleurs un tampon (50) réalisé dans une matière élastique, un logement d'extrémité creux (51) comportant un fond et un capuchon creux (65) comportant un fond et ayant une élasticité,
le tampon (50) est monté de manière étanche dans l'extrémité cylindrique (5) du cylindre extérieur (1A) de la chambre antérieure de manière à obturer l'extrémité cylindrique (5) de manière étanche aux liquides,
un distance (P) entre l'orifice d'extrémité (17) de l'extrémité cylindrique (5) et le tampon (50) étant calculée de manière qu'elle soit plus longue qu'une distance (Q) comprise entre la gorge de dérivation (3) et une surface d'extrémité du piston plongeur (7) sur le côté de la gorge de dérivation (3),
une extrémité (53) de l'extrémité cylindrique (5) est enclenchée de manière hermétique aux gaz dans la partie de raccordement du logement d'extrémité creux (51) comportant un fond, de manière qu'une section creuse du logement d'extrémité (51) située entre l'orifice d'extrémité (17) de l'extrémité cylindrique (5) et une surface intérieure d'un fond (55) du logement d'extrémité (51) soit conformée en chambre de dérivation (57),
une longueur (R) de la chambre de dérivation (57) est déterminée de manière qu'elle soit plus longue qu'une épaisseur (S) du tampon (50),
l'ouverture d'extrémité (17) est incluse dans une surface qui est en contact avec la chambre de dérivation (57),
plusieurs dérivations (59) sont creusées longitudinalement dans le logement d'extrémité (51) sur une paroi intérieure du logement d'extrémité (51),
plusieurs gorges (60) situées au fond et qui sont raccordées aux dérivations (59) sont creusées radialement sur une surface intérieure du fond (55) du logement d'extrémité (51),
un chambrage (61) est réalisé dans le logement à l'intérieur d'une partie dans laquelle sont concentrées toutes les gorges (60) situées au fond de manière à raccorder toutes les gorges (60) situées au fond au chambrage (61) du logement,
une partie (63) de montage d'une aiguille est suspendue à une surface extérieure du fond (55) du logement (51),
un petit trou (64) allant d'une extrémité (66) du support d'aiguille (63) au chambrage (61) du logement est creusé dans la partie (63) de montage de l'aiguille, et
la partie (63) de montage de l'aiguille est isolée de manière hermétique aux liquides par la section creuse du capuchon creux (65) qui comporte un fond.

4. Seringue à chambre double pouvant être préalablement emplie selon la revendication 3,
caractérisée
en ce qu'une surface de paroi extérieure de la partie (63) de montage de l'aiguille a un diamètre qui diminue progressivement vers son extrémité sous forme d'une surface conique (26) et
une surface de paroi intérieure du capuchon creux (65) comportant un fond comporte la même surface conique que ladite surface conique (26).

5. Seringue à chambre double pouvant être préalablement emplie selon la revendication 3,
caractérisée
en ce que la seringue à chambre double pouvant être préalablement emplie comprend par ailleurs un tube (71) formant une aiguille ainsi qu'un capuchon (65, 74),
la surface de paroi extérieure de la partie (63) de montage de l'aiguille a un diamètre qui diminue progressivement vers son extrémité en formant une surface conique,
une aiguille de base (72) du tube (71) formant une aiguille est perforée de part en part, de l'extrémité du petit trou (64) de la partie (63) de montage de l'aiguille jusqu'au chambrage (61) du logement, de sorte que l'aiguille de base (72) est fixée de manière étanche aux gaz dans le petit trou (64) de la partie (63) de montage de l'aiguille,
un trou d'aiguille (73), qui est foré à travers le tube (71) formant une aiguille, est raccordé au chambrage (61) du logement,
une aiguille d'extrémité (75) du tube (71) formant une aiguille est prévue de manière à être saillante sur la partie (63) de montage de l'aiguille,
le capuchon (65, 74) agit en protecteur de l'aiguille,
une longueur (K) de la section creuse de la protection de l'aiguille (74) est calculée de manière à être plus longue qu'une longueur totale (J) formée de la somme de la longueur de l'aiguille d'extrémité (75) et de la longueur de la partie (63) de montage de l'aiguille,
une paroi intérieure de la protection de l'aiguille (65, 74) comporte la même surface conique que la surface conique de la partie (63) de montage de l'aiguille, et
l'aiguille d'extrémité (75) est isolée hermétiquement dans la section creuse de la protection de l'aiguille (65, 74).

6. Seringue à chambre double pouvant être préalablement emplie selon la revendication 4,
caractérisée en ce que
la seringue à chambre double pouvant être préalablement emplie comprend par ailleurs un verrou (86) ayant une partie en forme de colonne (85),
le verrou (86) est suspendu à la surface extérieure (62) du fond (55) du logement,
la partie (85) en forme de colonne inclut la partie (63) de montage de l'aiguille,
un taraudage (88) étant réalisé sur une surface périphérique intérieure de la partie en forme de colonne (85),
un collet (80) est réalisé sur une surface de paroi extérieure du capuchon (65), et
le collet (80) est en prise par le taraudage (88) avec le verrou (86).
